# EUROPÄISCHE PATENTSCHRIFT

(11) **EP 0 533 013 B1**
(45) Veröffentlichungstag und Bekanntmachung des Hinweises auf die Patenterteilung: **18.10.1995**
(21) Anmeldenummer: 92115273.2
(22) Anmeldetag: 07.09.1992
(51) Int. Cl.: C07C 61/15, C07C 51/09, C07C 69/74, C07C 67/42

(54) **Verfahren zur Herstellung von 1-Fluor-cyclopropan-1-carbonsäure**
Process for the production of 1-fluor-cyclopropane-1-carboxylic acid
Procédé de préparation de l'acide 1-fluoro cyclopropane-1-carboxylique

(30) Priorität: 19.09.1991 DE 4131139
(43) Veröffentlichungstag der Anmeldung: 24.03.1993
(73) Patentinhaber: BAYER AG, 51368 Leverkusen (DE)
(72) Erfinder: Böhm, Stefan, Dr., W-5090 Leverkusen 1 (DE); Marhold, Albrecht, Dr., W-5090 Leverkusen 1 (DE)

(56) Entgegenhaltungen:
- EP-A- 0 436 348

## Beschreibung

Die vorliegende Erfindung betrifft ein neues Verfahren zur Herstellung der bekannten 1-Fluor-cyclopropan-1-carbonsäure, die als Zwischenprodukt zur Synthese von Wirkstoffen mit fungiziden Eigenschaften verwendet werden kann.

Es ist bereits bekannt geworden, daß sich 1-Fluor-cyclopropan-1-carbonsäure durch Oxidation von 1-Fluor-cyclopropyl-methyl-keton herstellen läßt (vgl. EP-OS 0 436 348). Nachteilig an diesem Verfahren ist jedoch, daß die als Ausgangsprodukt benötigte Verbindung nur durch mehrstufige Synthese zugänglich ist und die Ausbeuten bei den einzelnen Reaktionsschritten nur relativ gering sind.

Es wurde nun gefunden, daß man 1-Fluor-cyclopropan-1-carbonsäure der Formel 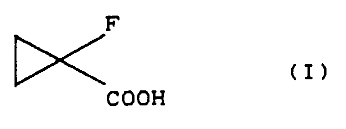
erhält, wenn man
a) in einer ersten Stufe 1-Fluor-cyclopropyl-phenylketone der Formel 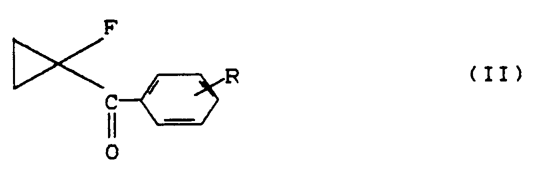 in welcher
   - R: für Wasserstoff, Halogen, Methyl, Methoxy, Phenyl oder Phenoxy steht,
   mit Peroxy-Verbindungen in Gegenwart eines Verdünnungsmittels umsetzt und
b) in einer zweiten Stufe die entstandenen 1-Fluor-cyclopropan-1-carbonsäure-ester der Formel 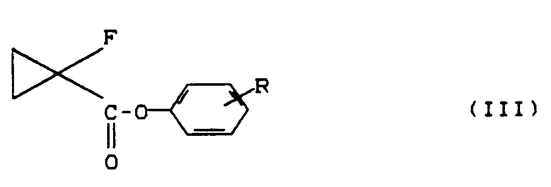 in welcher
   - R: die oben angegebene Bedeutung hat,
   mit Basen in Gegenwart eines Verdünnungsmittels umsetzt und danach ansäuert.

Es ist als äußerst überraschend anzusehen, daß sich 1-Fluor-cyclopropan-1-carbonsäure nach dem erfindungsgemäßen Verfahren in glatter Reaktion mit hoher Ausbeute herstellen läßt. Aufgrund des bekannten Standes der Technik war nämlich damit zu rechnen, daß im Zuge der Behandlung mit Peroxy-Verbindungen nicht der Phenyl-Ring, sondern der Cyclopropyl-Rest bei der Umlagerung wandern würde und daher Cyclopropylester statt Phenylester entstehen würden. Unerwartet ist auch, daß der fluorierte Cyclopropyl-Rest während der Umsetzung nicht in nennenswertem Maße angegriffen wird.

Das erfindungsgemäße Verfahren zeichnet sich durch eine Reihe von Vorteilen aus. So ermöglicht es die Herstellung der 1-Fluorcyclopropan-1-carbonsäure in sehr guten Ausbeuten und hoher Reinheit. Ferner sind auch die benötigten Ausgangssubstanzen in einfacher Weise und in größeren Mengen zugänglich.

Verwendet man 1-Fluor-cyclopropyl-(4-chlor-phenyl)-keton als Ausgangssubstanz, m-Chlorperbenzoesäure als Peroxy-Verbindung, Natriumhydroxid als Base und Salzsäure als Acidifizierungsmittel, so kann der Verlauf des erfindungsgemäßen Verfahrens durch das folgende Formelschema veranschaulicht werden: 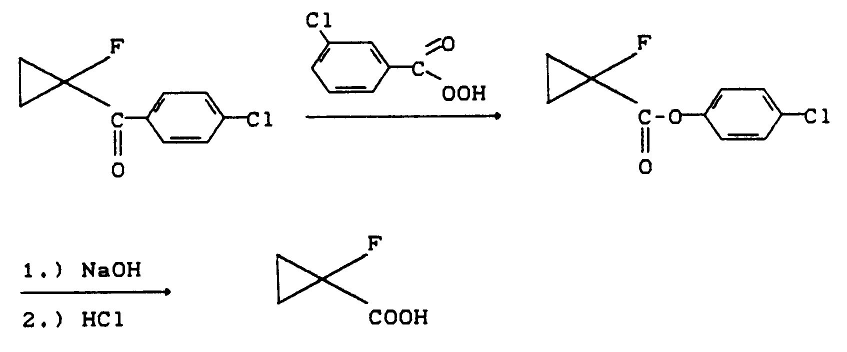
Die bei der Durchführung des erfindungsgemäßen Verfahrens als Ausgangssubstanzen benötigten 1-Fluor-cyclopropyl-phenyl-ketone sind durch die Formel (II) allgemein definiert. In dieser Formel steht
- R: für Wasserstoff, Fluor, Chlor, Brom, Methyl, Methoxy, Phenyl oder Phenoxy.

Besonders bevorzugt sind diejenigen Verbindungen der Formel (II), in denen
- R: für Wasserstoff steht oder
- R: in 4-Position gebunden ist und für Fluor, Chlor, Brom, Methyl, Methoxy, Phenyl oder Phenoxy steht.

Die 1-Fluor-cyclopropyl-phenyl-ketone der Formel (II) sind bekannt oder lassen sich nach prinzipiell bekannten Verfahren in einfacher Weise herstellen (vgl. EP-OS 0 180 136).

Als Peroxy-Verbindungen kommen bei der Durchführung der ersten Stufe des erfindungsgemäßen Verfahrens alle für derartige Reaktionen üblichen organischen und anorganischen Stoffe mit einer Peroxid-Gruppierung in Frage. Vorzugsweise verwendbar sind Persäuren, wie Perbenzoesäure, m-Chlor-perbenzoesäure, Peressigsäure, Perpropionsäure und Peroxy-trifluoressigsäure, ferner Bortrifluorid-Wasserstoffperoxid und Kaliumperoxydisulfat im Gemisch mit Schwefelsäure.

Die in Betracht kommenden Peroxy-Verbindungen sind bekannt.

Die bei der Durchführung der ersten Stufe des erfindungsgemäßen Verfahrens anfallenden 1-Fluor-cyclopropan-1-carbonsäureester der Formel (III) sind neu.

Als Basen kommen bei der Durchführung der zweiten Stufe des erfindungsgemäßen Verfahrens alle für derartige Verseifungen üblichen anorganischen Hydroxy-Verbindungen in Frage. Vorzugsweise verwendbar sind Alkalimetallhydroxide, wie Natriumhydroxid und Kaliumhydroxid, und außerdem Erdalkalimetallhydroxide, wie Calciumhydroxid.

Zur Acidifizierung können bei der Durchführung der zweiten Stufe des erfindungsgemäßen Verfahrens alle für derartige Umsetzungen üblichen organischen und anorganischen Säuren verwendet werden. Vorzugsweise verwendbar ist Salzsäure.

Als Verdünnungsmittel kommen bei der Durchführung der ersten Stufe des erfindungsgemäßen Verfahrens vorzugsweise halogenierte aliphatische Kohlenwasserstoffe, gegebenenfalls halogenierte aromatische Kohlenwasserstoffe sowie aliphatische Carbonsäuren in Betracht. Beispielhaft genannt seien Methylenchlorid, Dichlorethan, Chloroform, Tetrachlorkohlenstoff, Benzol, Toluol, Chlorbenzol, Essigsäure und Propionsäure.

Die Reaktionstemperaturen können bei der Durchführung der ersten Stufe des erfindungsgemäßen Verfahrens innerhalb eines bestimmten Bereiches variiert werden. Im allgemeinen arbeitet man bei Temperaturen zwischen -10°C und 120°C, vorzugsweise zwischen 0°C und 100°C.

Bei der Durchführung der ersten Stufe des erfindungsgemäßen Verfahrens arbeitet man im allgemeinen unter Normaldruck. Es ist aber auch möglich, unter erhöhtem oder vermindertem Druck zu arbeiten.

Bei der Durchführung der ersten Stufe des erfindungsgemäßen Verfahrens setzt man auf 1 Mol an 1-Fluor-cyclopropyl-phenyl-keton der Formel (II) im allgemeinen 1 bis 3 Mol, vorzugsweise 1,2 bis 2,5 Mol an Peroxy-Verbindung ein. Die Aufarbeitung erfolgt nach üblichen Methoden. Im allgemeinen geht man so vor, daß man gegebenenfalls nach vorheriger Kühlung den festen Rückstand absaugt, die organische Phase nacheinander mit wäßriger Natriumsulfit-Lösung, mit verdünnter wäßriger Natronlauge und wäßriger Natriumchlorid-Lösung wäscht, dann trocknet und einengt.

Als Verdünnungsmittel kommen bei der Durchführung der zweiten Stufe des erfindungsgemäßen Verfahrens vorzugsweise Gemische aus Wasser und Ether in Betracht. Bevorzugt verwendbare Ether sind dabei Diethylether, Methyl-tert.-butyl-ether, Tetrahydrofuran und Dioxan.

Die Reaktionstemperaturen können auch bei der Durchführung der zweiten Stufe des erfindungsgemäßen Verfahrens innerhalb eines bestimmten Bereiches variiert werden. Im allgemeinen arbeitet man bei Temperaturen zwischen -5°C und +100°C, vorzugsweise zwischen 0°C und 60°C.

Auch bei der Durchführung der zweiten Stufe des erfindungsgemäßen Verfahrens arbeitet man im allgemeinen unter Normaldruck. Es ist jedoch auch möglich, unter erhöhtem oder vermindertem Druck zu arbeiten.

Bei der Durchführung der zweiten Stufe des erfindungsgemäßen Verfahrens setzt man auf 1 Mol an 1-Fluor-cyclopropan-1-carbonsäure-ester der Formel (III) im allgemeinen eine äquimolare Menge an Base ein. Es ist jedoch auch möglich, die Base nur in katalytischen Mengen oder auch in einer mehr als äquimolaren Menge einzusetzen. In einer speziellen Variante kann die Esterspaltung auch mit Hilfe von Säuren durchgeführt werden. - Die Aufarbeitung erfolgt wiederum nach üblichen Methoden. Im allgemeinen geht man so vor, daß man das Reaktionsgemisch mit einem in Wasser wenig löslichen organischen Solvens extrahiert, die wäßrige Phase gegebenenfalls unter Kühlung ansäuert und das entstehende Gemisch mit einem in Wasser wenig löslichen organischen Solvens extrahiert und schließlich diese organische Phase trocknet und einengt. Das entstehende Produkt kann gegebenenfalls nach üblichen Methoden von eventuell noch enthaltenen Verunreingungen befreit werden.

Die 1-Fluor-cyclopropan-1-carbonsäure der Formel (I) ist ein wertvolles Zwischenprodukt zur Synthese von Wirkstoffen mit fungiziden Eigenschaften (vgl. EP-OS 0 436 348 und EP-OS 0 297 345). So läßt sich zum Beispiel die Verbindung der Formel 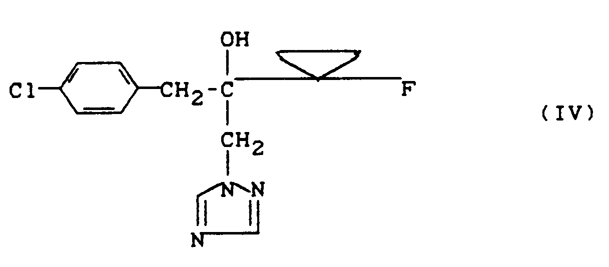
herstellen, indem man 1-Fluor-cyclopropan-1-carbonsäure der Formel 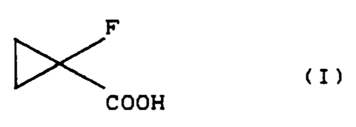
mit Hilfe von Thionylchlorid in das Säurechlorid der Formel 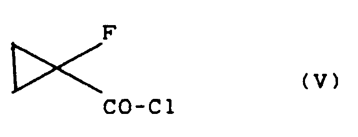
überführt, dieses dann mit 4-Chlorbenzyl-zinkchlorid der Formel 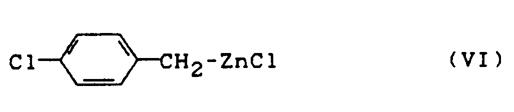
umsetzt und das entstehende Keton der Formel 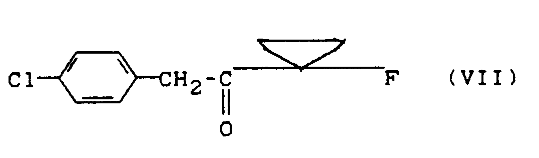
mit Dimethylsulfonium-methylid der Formel 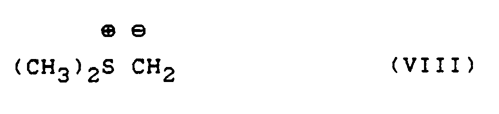
zur Reaktion bringt und das dabei gebildete Oxiran der Formel 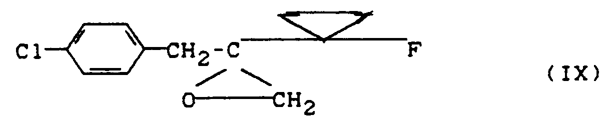
mit 1,2,4-Triazol umsetzt.

Die Durchführung des erfindungsgemäßen Verfahrens wird durch folgende Beispiele veranschaulicht.

### Beispiel 1

a) Herstellung von 1-Fluor-cyclopropan-1-carbonsäure-(4-chlorphenyl)-ester der Formel 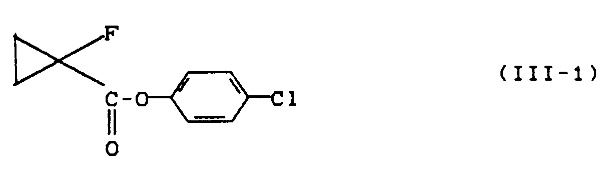 Eine Lösung von 130 g (0,65 Mol) 1-Fluor-cyclopropyl-(4-chlorphenyl)-keton in 1300 ml Chloroform wird bei Raumtemperatur mit 225 g (0,9 Mol) m-Chlorperbenzoesäure (70 %ig) versetzt. Die Mischung wird 24 Stunden unter Rückfluß gerührt. Dann gibt man weitere 120 g (0,48 Mol) m-Chlorperbenzoesäure (70 %ig) hinzu und rührt nochmals 24 Stunden unter Rückfluß. Anschließend wird das Reaktionsgemisch mit Eis gekühlt, der entstandene Niederschlag wird abgesaugt und mehrfach mit Chloroform gewaschen. Die vereinigten organischen Lösungen werden nacheinander mit 10 %iger wäßriger Natriumsulfit-Lösung, zweimal mit 5 %-iger wäßriger Natronlauge und mit verdünnter wäßriger Natriumchlorid-Lösung gewaschen. Nach dem Trocknen über Magnesiumsulfat wird die organische Phase durch Abziehen des Lösungsmittels unter vermindertem Druck eingeengt. Man erhält auf diese Weise 104 g (75 % der Theorie) an
   1-Fluor-cyclopropan-1-carbonsäure-(4-chlorphenyl)-ester in Form eines Öles.
   - ¹⁹F-NMR (188 MHz, CDCl₃/CFCl₃):: δ = 198,2 ppm
   - ¹H-NMR (200 MHz, CDCl₃/TMS):: δ = 1,48-1,62 ppm (m, 4H, cyclopropyl-H), 7,08 und 7,34 ppm (AA'BB'-System, 4 H, arom.-H).
b) Herstellung von 1-Fluor-cyclopropan-1-carbonsäure der Formel 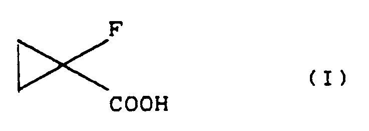 Eine Lösung von 138 g (0,64 Mol) 1-Fluor-cyclopropan-1-carbonsäure-(4-chlorphenyl)-ester in 250 ml Methyl-tert.-butyl-ether wird bei 0°C mit einer Lösung von 25,6 g (0,64 Mol) Natriumhydroxid in 250 ml Wasser versetzt. Man rührt 3 Stunden bei Raumtemperatur und extrahiert anschließend mit Methyl-tert.-butyl-ether 18 Stunden im Perforator. Die wäßrige Phase wird unter Eiskühlung mit 150 ml konzentrierter Salzsäure angesäuert und danach dreimal mit je 300 ml Diethylether extrahiert. Nach dem Trocknen über Magnesiumsulfat wird diese organische Phase durch Abziehen des Lösungsmittels unter vermindertem Druck eingeengt. Man erhält auf diese Weise 52 g (78 % der Theorie) an 1-Fluor-cyclopropan-1-carbonsäure in Form einer Festsubstanz vom Schmelzpunkt 66-68°C.
   - ¹⁹F-NMR (188 MHz, CDCl₃/CFCl₃):: δ = 199 ppm
   - ¹H-NMR (200 MHz, CDCl₃/TMS):: δ = 1,40-1,52 ppm (m, 4H, cyclopropyl-H), 9,25 ppm (1H, COOH).
c) Herstellung des als Ausgangsprodukt benötigten 1-Fluor-cyclopropyl-(4-chlorphenyl)-ketons der Formel 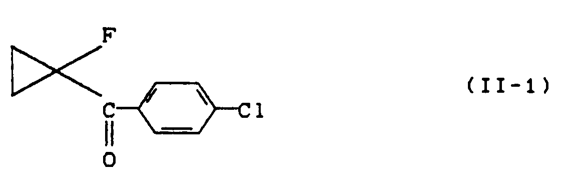 2000 g (34,5 Mol) Kaliumfluorid werden in 7 Litern Diethylenglycol vorgelegt und bei Raumtemperatur mit 500 ml Toluol versetzt. Nach Abdestillieren des Toluols bei 150°C läßt man das Gemisch auf 100°C abkühlen, fügt 1500 g (5 Mol) 4-Chlor-phenyl-1-brom-3-chlor-n-propyl-keton portionsweise innerhalb von 15 Minuten hinzu. Das Reaktionsgemisch wird 4 Stunden auf 150°C erhitzt, danach abgekühlt und auf 10 Liter Wasser gegossen. Man extrahiert das entstandene Gemisch fünfmal mit je 2 Liter Toluol, wäscht die vereinigten organischen Phasen mit Wasser, trocknet über Magnesiumsulfat und engt durch Abziehen des Lösungsmittels unter vermindertem Druck ein. Der verbleibende Rückstand wird im Hochvakuum bdestilliert, und das aufgefangene Destillat wird im Hochvakuum über eine Drehbandkolonne fraktioniert destilliert. Man erhält auf diese Weise 100 g (10 % der Theorie) an 1-Fluor-cyclopropyl-(4-chlorphenyl)-keton in Form einer Flüssigkeit vom Siedepunkt 70-76°C bei 0,03 mbar.
   - ¹⁹F-NMR (188 MHz, CDCl₃/CFCl₃):: δ = 191,7 ppm
   - ¹H-NMR (200 MHz, CDCl₃/TMS);: δ = 1,39-1,62 ppm (m, 4H, Cyclopropyl-H), 7,44 und 7,96 ppm (AA'BB'-System, 4H, arom.-H).

### Verwendungsbeispiel

Herstellung der Verbindung der Formel 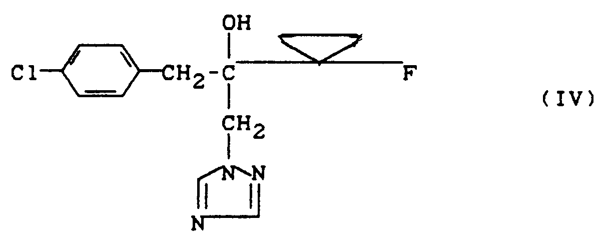
a) Herstellung der Verbindung der Formel 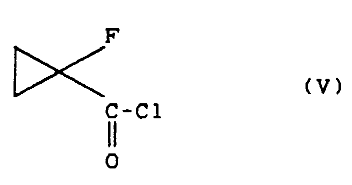 52 g (0,5 Mol) 1-Fluorcyclopropan-carbonsäure werden bei Raumtemperatur mit 3 Tropfen Dimethylformamid und 71 g (0,6 Mol) Thionylchlorid versetzt. Das Gemisch wird unter Rühren langsam auf Rückflußtemperatur erhitzt und 2 Stunden bei dieser Temperatur gerührt. Anschließend wird das Gemisch einer fraktionierten Destillation unterworfen. Man erhält auf diese Weise 42,8 g (70 % der Theorie) an 1-Fluorcyclopropan-carbonsäurechlorid in Form einer Flüssigkeit vom Siedepunkt 71°C bei 300 mbar.
   - ¹⁹F-NMR (188 MHz, CDCl₃/CFCl₃):: δ = 187,4 ppm
   - ¹H-NMR (200 MHz, CDCl₃/TMS):: δ = 1,58-1,73 ppm (m, Cyclopropyl-H).
b) Herstellung der Verbindung der Formel 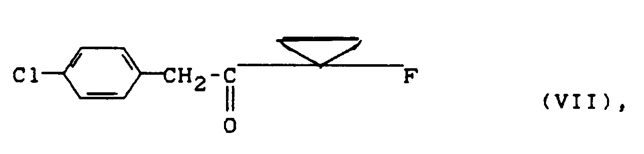 In eine Lösung von 12,5 g (55 mMol) 4-Chlorbenzylzinkchlorid in 60 ml absolutem Dimethoxyethan werden bei Raumtemperatur 0,018 g Bis-(triphenylphosphin)-palladium(II)chlorid und 6,1 g (50 mMol) 1-Fluor-cyclopropan-carbonsäurechlorid gegeben. Das Gemisch wird unter Rühren und unter Stickstoffatmosphäre 30 Minuten unter Rückfluß erhitzt. Danach wird das Reaktionsgemisch mit Toluol und verdünnter, wäßriger Salzsäure versetzt. Die organische Phase wird abgetrennt, zweimal mit Wasser gewaschen, über Natriumsulfat getrocknet und durch Abziehen des Lösungsmittels unter vermindertem Druck eingeengt. Der verbleibende Rückstand wird bei einer Badtemperatur von 70°C unter einem Druck von etwa 0,05 mbar andestilliert. Man erhält auf diese Weise 9,6 g eines gelblichen Feststoffes, der gemäß Gaschromatogramm zu 70 % aus 4-Chlorbenzyl-1-fluorcyclopropyl-keton besteht. Die Ausbeute errechnet sich danach zu 63 % der Theorie. Nach Umkristallisation aus einem Gemisch von Hexan und Ethanol erhält man 3,3 g an 4-Chlorbenzyl-1-fluorcyclopropyl-keton in Form eines gelblichen Feststoffes vom Schmelzpunkt 53-54°C.
c) Herstellung der Verbindung der Formel 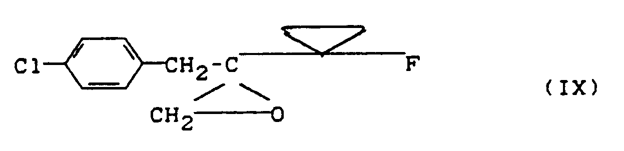 In ein Gemisch aus 2,2 g (17,1 mMol) Trimethylsulfoxoniumchlorid und 3,3 g (15,5 mMol) 4-Chlorbenzyl-(1-fluorcyclopropyl)-keton in 20 ml Toluol tropft man bei Raumtemperatur innerhalb einer Stunde 21 ml 45 %ige wäßrige Natronlauge ein Nach beendeter Zugabe wird 2 Stunden bei 40°C nachgerührt. Anschließend werden die Phasen getrennt, und die wäßrige Phase wird dreimal mit Toluol extrahiert. Die vereinigten organischen Phasen werden einmal mit Wasser gewaschen, über Natriumsulfat getrocknet und unter vermindertem Druck eingeengt. Der Rückstand wird ohne zusätzliche Reinigung für die weitere Umsetzung verwendet.
d) Herstellung der Verbindung der Formel 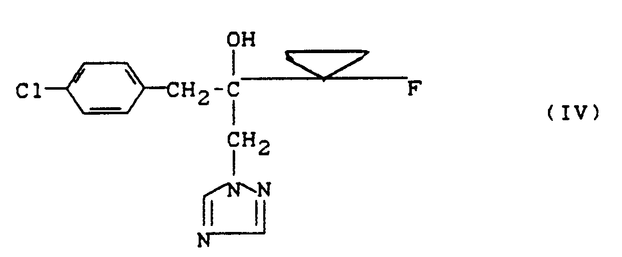 eine Lösung von 3,3 g (15,7 mMol) 2-(4-Chlorbenzyl)-2-(1-fluorcyclopropyl)-oxiran in 10 ml Dimethylformamid wird bei 80°C unter Rühren in ein Gemisch aus 3,3 g (47,1 mMol) 1,2,4-Triazol und 0,35 g (3,14 mMol) Kalium-tert.-butylat in 20 ml Dimethylformamid eingetropft. Nach beendeter Zugabe wird das Reaktionsgemisch 13 Stunden bei 80°C gerührt. Anschließend wird das Lösungsmittel unter vermindertem Druck abgezogen, und der verbleibende Rückstand wird in Wasser aufgenommen. Die wäßrige Phase wird viermal mit Dichlormethan extrahiert. Die vereinigten organischen Phasen werden über Natriumsulfat getrocknet und danach unter vermindertem Druck eingeengt. Der verbleibende Rückstand wird mit dem Laufmittel Cyclohexan: Ethylacetat = 2:1 an Kieselgel chromatographiert. Nach dem Eindampfen des Eluates erhält man 2,2 g (47 % der Theorie) an 1-(4-Chlorphenyl)-2-(1-fluorcyclopropyl)-3-(1,2,4-triazol-1-yl)-propan-2-ol in Form einer Festsubstanz.
   - ¹H-NMR (200 MHz, CDCl₃):: δ = 0,0-0,8 (m, 4H); 3,0 (AB-System, 2H); 4,01 (s, 1H, OH); 4,3 (AB-System, 2H); 7,28 (s, 4H); 7,98 (s, 1H); 8,13 (s, 1H).

## Patentansprüche

1. Verfahren zur Herstellung von 1-Fluor-cyclopropan-1-carbonsäure der Formel 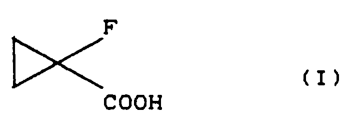 dadurch gekennzeichnet, daß man
a) in einer ersten Stufe 1-Fluor-cyclopropylphenyl-ketone der Formel 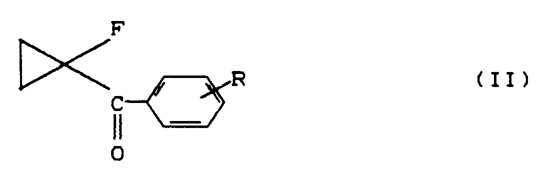 in welcher
R für Wasserstoff, Halogen, Methyl, Methoxy, Phenyl oder Phenoxy steht,
mit Peroxy-Verbindungen in Gegenwart eines Verdünnungsmittels umsetzt und
b) in einer zweiten Stufe die entstandenen 1-Fluor-cyclopropan-1-carbonsäure-ester der Formel 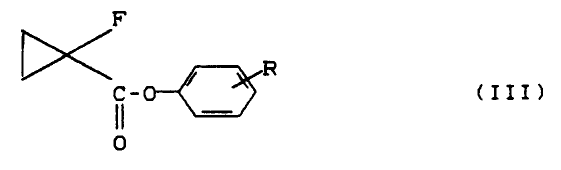 in welcher
R die oben angegebene Bedeutung hat,
mit Basen in Gegenwart eines Verdünnungsmittels umsetzt und danach ansäuert.

2. Verfahren gemäß Anspruch 1, dadurch gekennzeichnet, daß man als Ausgangssubstanzen 1-Fluor-cyclopropylphenyl-ketone der Formel (II) einsetzt, in denen
R für Wasserstoff, Fluor, Chlor, Brom, Methyl, Methoxy, Phenyl oder Phenoxy steht.

3. Verfahren gemäß Anspruch 1, dadurch gekennzeichnet, daß man als Peroxy-Verbindungen Perbenzoesäure, m-Chlor-perbenzoesäure, Peressigsäure, Perpropionsäure, Peroxy-trifluoressigsäure, Bortrifluorid-Wasserstoffperoxid oder ein Gemisch aus Kaliumperoxydisulfat und Schwefelsäure einsetzt.

4. Verfahren gemäß Anspruch 1, dadurch gekennzeichnet, daß man Erdalkalimetallhydroxide oder Alkalimetallhydroxide als Basen einsetzt.

5. Verfahren gemäß Anspruch 1, dadurch gekennzeichnet, daß man bei der Durchführung der ersten Stufe halogenierte aliphatische Kohlenwasserstoffe, gegebenenfalls halogenierte aromatische Kohlenwasserstoffe oder aliphatische Carbonsäuren als Verdünnungsmittel einsetzt.

6. Verfahren gemäß Anspruch 1, dadurch gekennzeichnet, daß man bei der Durchführung der zweiten Stufe Gemische aus Wasser und Ether als Verdünnungsmittel einsetzt.

7. Verfahren gemäß Anspruch 1, dadurch gekennzeichnet, daß man bei der Durchführung der ersten Stufe bei Temperaturen zwischen -10°C und 120°C und bei der Durchführung der zweiten Stufe bei Temperaturen zwischen -5°C und +100°C arbeitet.

8. 1-Fluor-cyclopropan-1-carbonsäureester der Formel 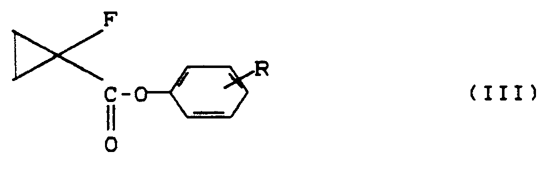 in welcher
R für Wasserstoff, Halogen, Methyl, Methoxy, Phenyl oder Phenoxy steht.

## Claims

1. Process for the preparation of 1-fluoro-cyclopropane-1-carboxylic acid, of the formula 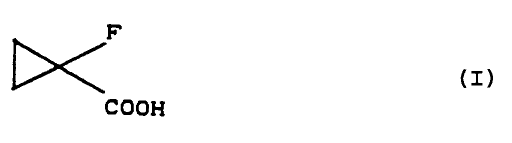 characterised in that
a) in a first step, 1-fluoro-cyclopropyl phenyl ketones of the formula 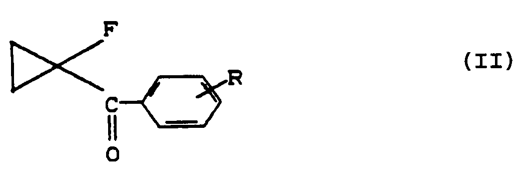 in which
R represents hydrogen, halogen, methyl, methoxy, phenyl or phenoxy,
are reacted with peroxy compounds in the presence of a diluent, and
b) in a second step, the resulting 1-fluorocyclopropane-1-carboxylates of the formula 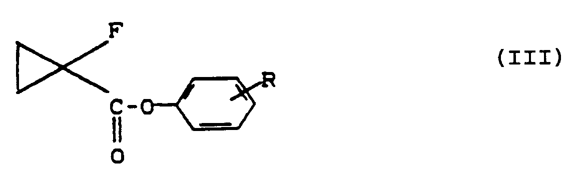 in which
R has the abovementioned meaning,
are reacted with bases in the presence of a diluent, and the reaction mixture is then acidified.

2. Process according to Claim 1, characterised in that 1-fluoro-cyclopropyl phenyl ketones of the formula (II) in which
R represents hydrogen, fluorine, chlorine, bromine, methyl, methoxy, phenyl or phenoxy,
are employed as starting substances.

3. Process according to Claim 1, characterised in that perbenzoic acid, m-chloro-perbenzoic acid, peracetic acid, perpropionic acid, peroxy-trifluoroacetic acid, boron trifluoride/hydrogen peroxide or a mixture of potassium peroxydisulphate and sulphuric acid are employed as peroxy compounds.

4. Process according to Claim 1, characterised in that alkaline earth metal hydroxides or alkali metal hydroxides are employed as bases.

5. Process according to Claim 1, characterised in that halogenated aliphatic hydrocarbons, optionally halogenated aromatic hydrocarbons or aliphatic carboxylic acids are employed as diluents for carrying out the first step.

6. Process according to Claim 1, characterised in that mixtures of water and ethers are employed as diluents for carrying out the second step.

7. Process according to Claim 1, characterised in that the first step is carried out at temperatures between -10°C and 120°C and the second step is carried out at temperatures between -5°C and +100°C.

8. 1-Fluoro-cyclopropane-1-carboxylates of the formula 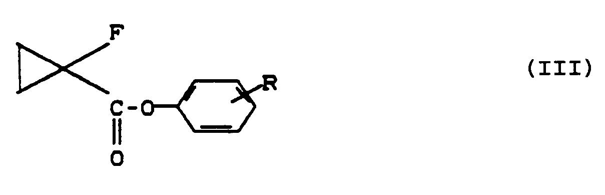 in which
R represents hydrogen, halogen, methyl, methoxy, phenyl or phenoxy.

## Revendications

1. Procédé de production de l'acide 1-fluoro-cyclopropane-1-carboxylique de formule 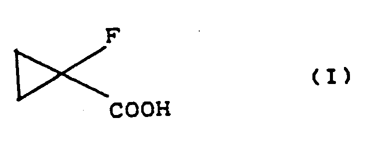 caractérisé en ce que
a) on fait réagir dans une première étape des 1-fluoro-cyclopropylphényl-cétones de formule 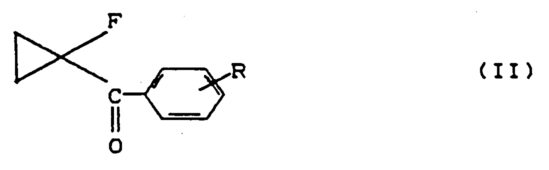 dans laquelle
R représente l'hydrogène, un halogène, un groupe méthyle, méthoxy, phényle ou phénoxy,
avec des composés peroxy, en présence d'un diluant, et
b) dans une seconde étape, on fait réagir l'ester d'acide 1-fluoro-cyclopropane-1-carboxylique produit, de formule 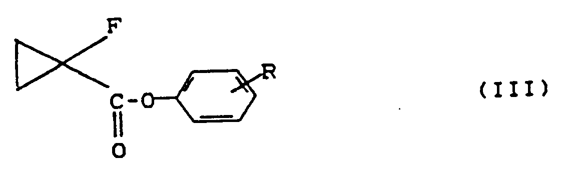 dans laquelle
R a la définition indiquée ci-dessus, avec des bases en présence d'un diluant, puis on acidifie.

2. Procédé suivant la revendication 1, caractérisé en ce qu'on utilise comme composés de départ des 1-fluoro-cyclopropylphényl-cétones de formule (II), dans lesquelles
R est de l'hydrogène, du fluor, du chlore, du brome, un groupe méthyle, méthoxy, phényle ou phénoxy.

3. Procédé suivant la revendication 1, caractérisé en ce qu'on utilise comme composés peroxy l'acide perbenzoïque, l'acide m-chloroperbenzoïque, l'acide peracétique, l'acide perpropionique, l'acide peroxytrifluoracétique, le peroxyde d'hydrogène combiné au trifluorure de bore ou un mélange de peroxydisulfate de potassium et d'acide sulfurique.

4. Procédé suivant la revendication 1, caractérisé en ce qu'on utilise comme base des hydroxydes de métaux alcalino-terreux ou des hydroxydes de métaux alcalins.

5. Procédé suivant la revendication 1, caractérisé en ce qu'on utilise comme diluants dans la conduite de la première étape, des hydrocarbures aliphatiques halogénés, des hydrocarbures aromatiques éventuellement halogénés ou des acides carboxyliques aliphatiques.

6. Procédé suivant la revendication 1, caractérisé en ce qu'on utilise comme diluant, dans la conduite de la seconde étape, des mélanges d'eau et d'éther.

7. Procédé suivant la revendication 1, caractérisé en ce qu'on opère à des températures comprises entre -10°C et 120°C et dans la conduite de la première étape et à des températures comprises entre -5°C et +100°C dans la conduite de la seconde étape.

8. Esters d'acide 1-fluoro-cyclopropane-1-carboxylique de formule 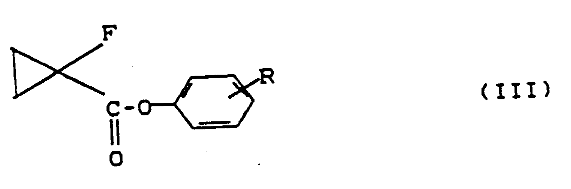 dans laquelle
R est de l'hydrogène, un halogène, un groupe méthyle, méthoxy, phényle ou phénoxy.
